# EUROPEAN PATENT APPLICATION

(11) **EP 4 431 288 A1**
(43) Date of publication of application: **18.09.2024**
(21) Application number: 24158603.1
(22) Date of filing: 20.02.2024
(51) Int. Cl.: B41J 2/045, B41J 2/32

(54) **METHOD FOR DELIVERING OR EJECTING A PREDETERMINED AMOUNT OF FLUID FROM A FLUID EJECTION HEAD**

(30) Priority: 14.03.2023 US 202318183472
(71) Applicant: Funai Electric Co., Ltd., Daito, Osaka, 574-0013 (JP)
(72) Inventor: GIBSON, Bruce D., Lexington, KY 40508 (US); JONES, Brian T., Lexington, KY 40508 (US); MARRA III, Michael A., Lexington, KY 40508 (US)
(74) Representative: Kurig, Thomas

(57) **Abstract**

A method for delivering a predetermined amount of fluid from a fluid ejection head (10) includes measuring one or more parameters selected from fluid flow parameters and electrical parameters for the fluid ejection head (10) to provide one or more measured parameters; calculating a fluid ejection offset value from the one or more measured parameters; storing the fluid ejection offset value in a memory location on the fluid ejection head (10); accessing the fluid ejection offset value by an ejection head controller (114); and adjusting an amount of fluid ejected from the fluid ejection head (10) during a fluid ejection step using the fluid ejection offset value to provide the predetermined amount of fluid from the fluid ejection head (10).

## Description

### TECHNICAL FIELD

The disclosure relates to fluid ejection devices and in particular to methods for providing accurate and predictable delivery of fluids using a fluid ejection device.

### BACKGROUND

Nasal spray devices have become important methods for delivering pharmaceutical drugs to patients. Such nasal spray devices are more convenient to use than the administration of drugs through IV or injection. Nasal spray devices also provide higher bioavailability of drugs compared to oral administration of drugs. The absorption of drugs through nasal spray devices is more rapid compared to the absorption of drugs administered orally since drugs delivered by nasal spray devices directly enter the blood stream making their effect more immediate.

### SUMMARY

### [Technical problem]

Conventional methods for delivering drugs via the nasal cavity include medicine droppers, multi-spray bottles with spray tips, single-dose syringes with spray tips, and dry powder systems. Some of the conventional methods for nasal drug delivery rely on pressurized containers to inject a mist of fluid into the nasal cavity. Accordingly, the drug delivery devices are typically designed for a specific drug and cannot be adapted to administer a different drug. Also, conventional spray type drug delivery devices are apt to provide significant errors in the administration of a set dosage of pharmaceutical fluids due to operator error and device variations.

Another area of the medical field that requires the precise administration of fluids is in the field of blood and sample analysis. A precise amount of an analytical fluid may need to be deposited on a glass slide or into the well of a micro-well plate in order to conduct analysis of a sample. Traditional methods of assay analyses use pipettes or elaborate electromechanical devices that can prepare multiple samples at a time. In an attempt to reduce the cost of such analytical devices and to provide more versatile drug delivery devices, inkjet technology has been adapted to be used for such applications.

With respect to ink jet technology used for a printing application, the droplet size can vary by +/- 18%. The droplet size variation primarily impacts the color gamut of the printed image. However, for medical applications, such as drug delivery or sample assay analysis, the volume of fluid delivered is critical and can affect the analysis or the dosage amount that is provided to a patient. The volume of fluid delivered depends on the number of droplets ejected at any given time and volume or size of each droplet. Accordingly, for a given number of fluid droplets, the variation in volume or size of the droplets provided by a conventional inkjet ejection head may be too high for the foregoing medical applications. Accordingly, there remains a need to improve the accuracy of the amount of fluid delivered by such fluid ejection devices.

### [Technical solutions]

In view of the foregoing, an embodiment of the disclosure provides a method for delivering a predetermined amount of fluid from a fluid ejection head. The method includes measuring one or more parameters selected from fluid flow parameters and electrical parameters for the fluid ejection head to provide one or more measured parameters; calculating a fluid ejection offset value from the one or more measured parameters; storing the fluid ejection offset value in a memory location on the fluid ejection head; accessing the fluid ejection offset value by an ejection head controller; and adjusting an amount of fluid ejected from the fluid ejection head during a fluid ejection step using the fluid ejection offset value to provide the predetermined amount of fluid from the fluid ejection head.

In some embodiments, the fluid flow parameters are selected from nozzle diameter, flow channel width, ejection chamber area, nozzle plate layer thickness, flow feature layer thickness, flow channel length, and combinations of two or more of the foregoing.

In some embodiments, the method further includes averaging one or more of the fluid flow parameters along the length of the fluid ejection head to provide a fluid flow parameter offset value.

In some embodiment, the method further includes storing the fluid flow parameter offset value in the memory location on the fluid ejection head.

In some embodiments, the electrical parameters are selected from heater sheet resistivity, heater sheet thickness, silicon nitride layer thickness, cavitation layer thickness, and a combination of two or more of the foregoing.

In some embodiments, the method further includes averaging one or more of the electrical parameters for the fluid ejection head to provide an electrical parameter offset value.

In some embodiments, the method further includes storing the electrical parameter offset value in a memory location on the fluid ejection head.

In some embodiments, the method further includes combining the fluid flow parameter offset value and the electrical parameter offset value to provide the fluid ejection offset value.

In some embodiments, the step of adjusting the amount of fluid ejected from the fluid ejection head is selected from changing a dropcount of fluid droplets ejected during the fluid ejection step and changing a size of fluid droplets ejected during the fluid ejection step.

In some embodiments, the size of fluid droplets ejected during a fluid ejection step is changed by a method selected from changing a fluid ejection temperature during the fluid ejection step, changing a firing pulse width of one or more fluid ejectors during the fluid ejection step, changing the voltage to the fluid ejection head, changing a firing frequency of one or more fluid ejectors during the fluid ejection step, and a combination of two or more of the foregoing.

In another embodiment, the disclosure provides a method for ejecting a predetermined amount of fluid from a fluid ejection head. The method includes measuring one or more parameters selected from the group consisting of fluid flow parameters and electrical parameters for the fluid ejection head to provide one or more measured parameters; calculating a fluid ejection offset value from the one or more measured parameters; storing the fluid ejection offset value in a memory location on the fluid ejection head; accessing the fluid ejection offset value by an ejection head controller; and adjusting a dropcount of fluid droplets ejected from the fluid ejection head during a fluid ejection step using the fluid ejection offset value to provide the predetermined amount of fluid ejected.

### [Technical effects]

An advantage of the embodiments of the disclosure is that a precise amount of fluid may be provided by a conventional fluid ejection head thereby greatly reducing the complexity and cost for devices designed to administer accurate dosages of fluids for medical analysis and pharmaceutical purposes.

### BRIEF DESCRIPTION OF THE DRAWINGS

FIG. 1 is a plan view, not to scale of a silicon wafer containing a plurality of fluid ejection heads.
FIG. 2 is a plan view, not to scale, of a single ejection head excised from the wafer of FIG. 1.
FIG. 3 is a partial, cross-sectional view, not to scale, of the ejection head of FIG. 2.
FIG. 4 is an enlarged plan view, not to scale, of a portion of the ejection head of FIG. 2.
FIG. 5 is a cross-sectional view, not to scale, of a nasal spray device for use with the methods of the disclosure.
FIG. 6 is a correlation graph between percent fluid volume change versus average percent change of the fluid flow channel width deviation from a nominal value.
FIG. 7 is a correlation graph between percent fluid volume change versus average percent change of the fluid flow feature layer thickness from a nominal value.

### DETAILED DESCRIPTION OF EMBODIMENTS

Ejection heads for fluid ejection devices are typically manufactured in stages in different manufacturing locations. While manufacturing tolerances for the components of the ejection heads are monitored closely, there are slight variations silicon wafer substrates and between ejection heads fabricated on the same silicon wafer substrate and also variation along a length of each ejection head that may result in a variation of the volume of fluid ejected relative to a predetermined volume that is required by a particular application. The methods described herein may be used to compensate for fluid ejection variations in order to improve the accuracy of the amount of fluid ejected from an ejection head.

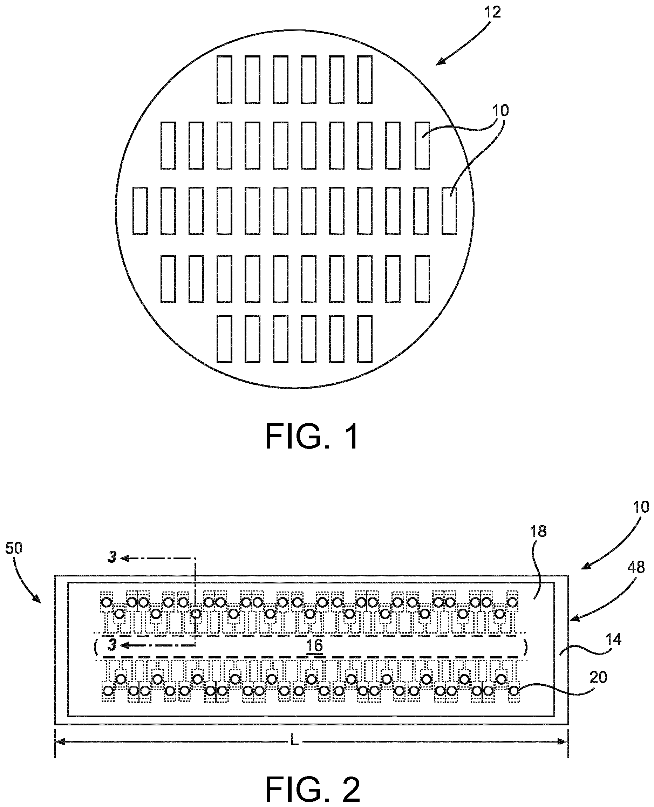

Multiple fluid ejection heads 10 are typically formed on a silicon wafer substrate 12 (FIG. 1) by depositing multiple layers of insulative, conductive and resistive materials as described in more detail below to provide fluid ejectors and circuits associated with the fluid ejectors. Once the foregoing layers are deposited on the silicon wafer substrate 12 to form the individual ejection heads 10, the wafer 12 is moved to another location for the deposition and fabrication of layers that provide flow features for each ejection head. A typical ejection head 10 as shown in FIG. 2 includes a silicon substrate 14 having a fluid supply via 16 etched therethrough, and a nozzle plate and a flow feature layer attached to the substrate 14 providing a plurality of nozzle holes 20 therein along a length L of the ejection head 10.

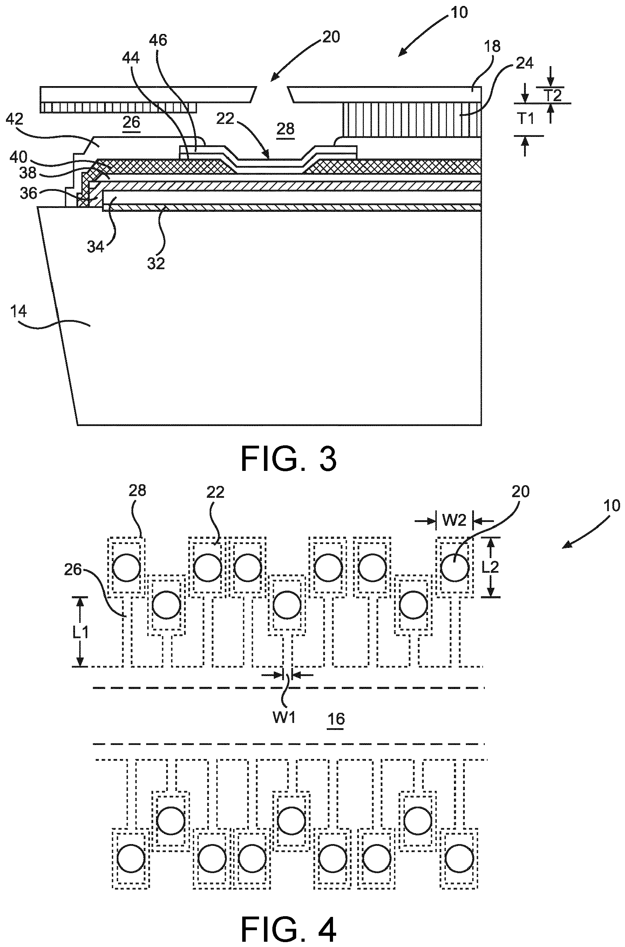

An enlarged view of a portion of the ejection head 10 is shown in FIGs. 3-4. Each ejection head 10 includes the silicon substrate 14 containing a plurality of fluid ejectors 22 deposited and associated electrical circuitry thereon and the fluid supply via 16 etched therethrough. Flow features are formed in the flow feature layer 24 and include a plurality of fluid flow channels 26 for providing fluid from the fluid supply via 16 to fluid ejection chambers 28. In some embodiments, the nozzle holes 20 are also formed in the flow feature layer 24. In other embodiments, the nozzle holes 20 for ejecting fluid therefrom are formed in a separate nozzle plate layer 18 attached to the flow feature layer 24.

With reference to FIG. 3, an enlarged cross-sectional view of a portion of the ejection head 10 is illustrated. A plurality of resistance and insulating layers are deposited on the silicon substrate 14 provided by the silicon substrate wafer 12 and include, but are not limited to, a first field oxide layer 32 formed adjacent to the silicon substrate 14, and an insulating layer 34 that may be a doped glass layer such as a phosphorus-doped silicon glass layer is deposited or grown on the field oxide layer 32. The field oxide layer 32 and insulating layer 34 have a combined thickness ranging from about 8,000 to about 30,000 Angstroms. A silicon nitride layer 36 is deposited on the insulating layer 34 as a passivation layer and underlies a portion of a resistance layer 38 so that the underlying portion is between the silicon substrate 14 and the resistance layer 38. The passivation layer 36 has a thickness ranging from about 1,000 to about 15,000 Angstroms. Layer 38 has a thickness ranging from about 1,500 to about 10,000 Angstroms (hereinafter "heater sheet thickness"). Next a metal conductive layer 40 such as a tantalum or aluminum is deposited on the resistance layer 38 so that the resistance layer 38 is totally encapsulated between the silicon nitride layer 36 and the conductive layer 40. The conductive layer 40 has a thickness ranging from about 2,000 to about 10,000 Angstroms. Finally, an encapsulating layer 42 that is selected from silicon oxide or silicon dioxide that is deposited on the conductive layer 40 by a chemical vapor deposition process from an organic silicon compound. The encapsulating layer 42 has a thickness ranging from about 2,000 to about 10,000 Angstroms. The encapsulating layer 42 is also resistant to etching and acts as an etch mask during the DRIE process for forming the fluid supply vias 16. The encapsulating layer 42 may also provide a planar surface for attaching the flow feature layer 24 containing the fluid flow channels 26 and fluid ejection chambers 28 thereto. In some embodiments, the fluid ejector 22 is further protected by a silicon nitride layer 44 having a thickness ranging from about 2000 to about 6000 Angstroms, and/or a tantalum cavitation layer 46 having a thickness ranging from about 2000 to about 6000 Angstroms.

During the processing of the silicon wafer 12 a resistivity of the resistance layer 38 (hereinafter "heater sheet resistivity") and a resistivity of cavitation layer 46 are measured at multiple locations on the wafer 12, and the thickness of layers 38 and 46 in the multiple locations are determined from the resistivity measurements of these layers. The thickness of layer 44 is determined by measuring a capacitance between layers 40 and 46 at multiple locations on the wafer 12 and calculating the thicknesses of layer 44 in the multiple locations from the capacitance measurements. The thickness of layers 38, 44 and 46 may also be determined by taking the resistance and capacitance measurements along the length L of the ejection head 10 from one end 48 to the other end 50 (FIG. 2). The values of the resistance and thicknesses of the layers (hereinafter "electrical parameters") may be stored individually on the ejection head 10 in a memory location thereon or a combination of two or more of the electrical parameters may be stored in the memory on the ejection head, in a memory or bar code on a fluid ejection cartridge, or in a memory on the fluid ejection device. A combination of the resistivity and/or thickness of one or more of the foregoing layers 32-46 is used to provide an electrical parameter offset value for each ejection head 10. The input energy to the ejection head is affected by the foregoing electrical parameters.

In the next step for making an ejection head, the wafer 12 containing multiple ejection heads 10 is transferred to a location for depositing a flow feature layer 24 thereon. The flow feature layer 24 is typically made of a photoimageable material such as a positive or negative photoresist material and has an overall thickness T1 ranging from about 12 to about 30 µm. The fluid ejection chambers 28 and fluid flow channels 26 are formed in the flow feature layer 24 using photoimaging and developing techniques. Next, a nozzle plate layer 18 is applied to the flow feature layer 24. Like the flow feature layer, the nozzle plate layer may be made of a photoresist material and the nozzle holes 20 are imaged and developed in the nozzle plate layer using photoimaging and developing techniques. The nozzle plate layer 18 has an overall thickness T2 ranging from about 12 to about 30 µm. Like the resistance, insulating and passivating layers described above, the flow feature layer thickness T1 and the nozzle plate layer thickness T2 may also vary along the length L of the ejection head 10 from end 48 to end 50 as described above.

As shown in FIG. 4, the fluid flow channels have a width W1 and a length L1 that may vary along the length L of the ejection head 10. The fluid flow channel width W1 may range from about 12 to about 40 µm. Likewise, each fluid ejection chamber has a length L2 and a width W2 that may vary along the length L of the ejection head 10 in the fluid flow layer 24. Each of the length L2 and width W2 of the fluid ejection chamber may range from about 20 to about 50 µm. Furthermore, each nozzle hole 20 formed in the nozzle plate layer 18 may have a diameter that varies along the length L of the ejection head 10. The nozzle hole diameter may range from about 15 to about 40 µm. Accordingly, one or more of width W1 and W2, length L1 and L2, thickness T1 and T2 or nozzle diameter may be averaged over the length of the ejection head 10 to provide a plurality of fluid flow parameter offset values or the fluid flow offset set values may be combined into a single fluid flow parameter offset value. The fluid flow parameter offset value(s) may be used alone or in combination with the electrical parameter offset value(s) to control the ejection of fluid from the ejection head 10.

All of the electrical parameter averages or offset values and fluid flow parameter averages or offset values may be stored in a memory location on each ejection head 10 or may be provided as separate values or combined as a single value in a memory on the ejection head, in a separate memory or bar code on a fluid ejection cartridge, or in a memory on the fluid ejection device.

Once the flow feature layer 24 and nozzle plate layer 18 are completed on the wafer for each ejection head 10, individual ejection heads 10 may be excised from the wafer and attached to a fluid ejection cartridge.

The following non-limiting example is provided to further illustrate the methods described herein.

### Electrical Parameters

During fabrication of CMOS and fluid ejectors, the following electrical parameters are measured:
1. Resistance layer 38 sheet resistivity at time of the wafter 12 fabrication;
2. Silicon nitride layer 44 thickness at time of the wafer 12 fabrication; and
3. Cavitation layer 46 resistivity at the time of wafer 12 fabrication.

Method A. The effect of each of the foregoing measured values 1, 2, and 3 on droplet size is calculated from data prepared from historical data and are stored separately as an offset value in a memory on the ejection head or on a fluid ejection cartridge containing the ejection head.

Method B. A composite effect from the measured values of 1, 2, and 3 on droplet size is calculated and recorded as a single electrical parameter offset value that reflects the combined drop size change. The single electrical parameter offset value is stored in a memory on the ejection head or on the fluid ejection cartridge.

Method C. The forgoing individual offset values from 1, 2, and 3 or the composite offset value may be saved to a spreadsheet for later combination with one or more offset values determined from the fluid flow parameters as set forth below.

### Fluid Flow Parameters

During the fabrication of the flow feature layer and nozzle plate layer, the following fluid flow parameters are measured optically for each ejection head along the length L of the ejection head.
4. Fluid flow layer thickness;
5. Length and width dimensions of each fluid flow channel;
6. Length and width dimension of each fluid ejection chamber;
7. Nozzle plate layer thickness; and
8. Nozzle diameter for each nozzle.

Method D. The effect of each of the foregoing measured values 4, 5, 6, 7, and 8 on droplet size is calculated from data prepared from historical data and are stored separately as an offset value in a memory on the ejection head or on a fluid ejection cartridge containing the ejection head.

Method E. A composite effect from the measured values of 4, 5, 6, 7, and 8 on droplet size is calculated and recorded as a single fluid flow parameter offset value that reflects the combined drop size change. The single fluid flow parameter offset value is stored in a memory on the ejection head or on the fluid ejection cartridge.

Method F. One or more of the individual offset values from 1, 2, and 3 or the composite electrical parameter offset value and one or more of the foregoing fluid flow parameter offset values from 4, 5, 6, 7, and 8 or the composite fluid flow parameter offset value may be combined into a single fluid ejection offset value that is used by the controller of the fluid ejection device to change the dropcount or the droplet size during a fluid ejection step.

In one embodiment, at the time of use of the fluid ejection device, the controller reads all of the separate values of 1-8 set forth above from a memory on the ejection head or fluid cartridge from Method A and Method D and calculates the composite effect of each of the separate values on the dropcount or droplet size and adjusts the dropcount or droplet size to provide the predetermine amount of fluid from the ejection head.

In another embodiment, at the time of use of the fluid ejection device, the controller reads the composite effect from Method B on droplet size from the values of 1, 2, and 3 from the memory, and the composite effect from Method E on droplet size from the measured values from 4, 5, 6, 7, and 8 from the memory and calculates a combined effect on dropcount or droplet size from the two separate composite effects.

In yet another embodiment, at the time of use of the fluid ejection device, the controller reads the single fluid offset value from Method F on dropcount or droplet size for use in selecting the dropcount or droplet size to deliver the predetermine amount of fluid.

The controller may adjust the droplet size from the calculated or read offset values set forth above, by changing the temperature of fluid to be ejected from the ejection head using an ejection head heater, changing the firing pulse of the fluid ejectors, changing the voltage to the ejection head, or changing the firing frequency of the fluid ejectors. The controller may change the dropcount by increasing or decreasing the total number of firing pulses for the fluid ejectors in order to eject the predetermined amount of fluid.

The following non-limiting example is provided to illustrate how to increase or decrease the total number of firing pulses in order to eject a predetermined amount of fluid based on average fluid flow parameter measurements.

### EXAMPLE

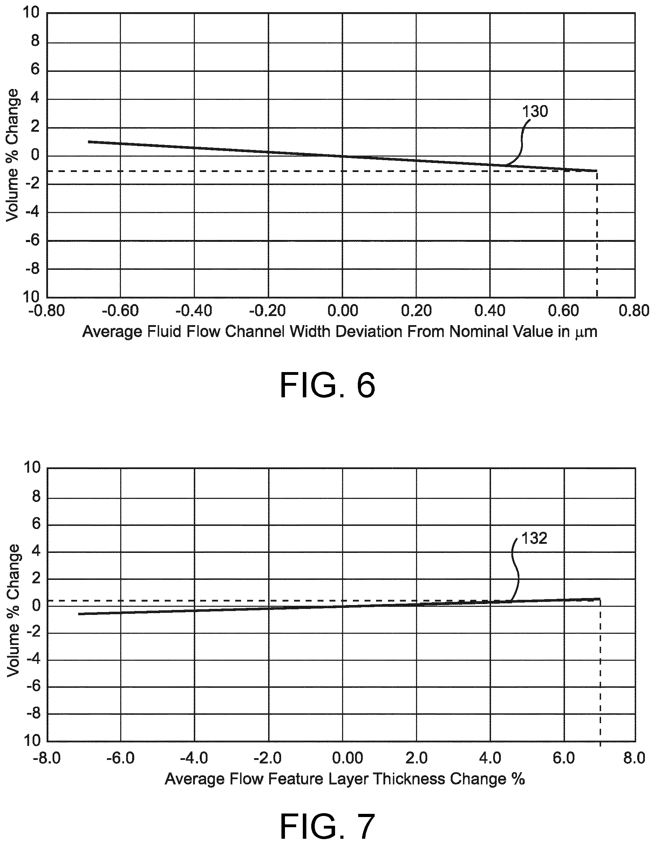

As described above, one or more of the fluid flow parameters may vary due to processing techniques. The variations may be in the form of dilation of the fluid flow parameters and thickness of the fluid flow feature layer which are measured independently across the length of the ejection head. The fluid flow channel width measurement is representative of the dilation that occurs during processing of the flow feature layer. The fluid flow channel width measurement is an average of all or a sampling of some of the fluid flow channel widths along the length of the ejection head. A correlation between dilation and droplet size is used as one of the drop size offset values for the ejection head. For example, if the average fluid flow channel width along the length of the ejection head measures 0.7µm wider than a predetermined fluid flow channel width represented by 0.00 on FIG. 6, then from FIG. 6, the offset value for fluid volume from line 130 is -0.95%. If the average thickness of the fluid flow feature layer is above a predetermined thickness (represented by 0.00) by 7.1% then a corresponding offset value for fluid volume from line 132 is 0.11% from the correlation in FIG. 7.

In order to compensate for the percent volume change due to the flow feature values determined above, the offset values are combined to provide an adjustment in the fluid droplet count. In this example the offset values are combined to give a composite adjustment of (100 - 0.95)*(100 + 0.11)/100 = 98.9%. The composite adjustment means that ejection head is expected to dispense a total fluid volume that is below a predetermined nominal value. In order to increase the dispense volume to the predetermined volume, the number of firing pulses is increased to 100*100/98.9 = 101.1% over the nominal number of firing pulses.

While the foregoing methods primarily describe using the fluid flow parameters for individual ejection heads 10, the fluid flow parameters obtained from wafer level processing may also be used. However, the adjustments to fluid delivery obtained from the wafer-level parameters may be less accurate than the adjustments made from the fluid flow parameters obtained from individual ejection heads on the wafer.

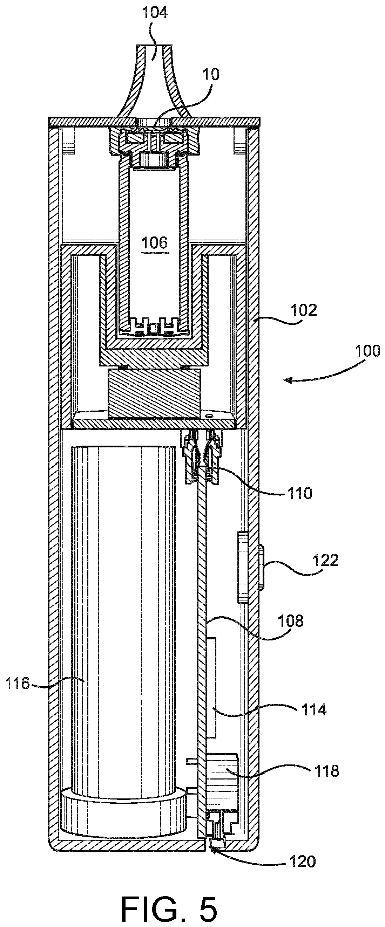

A representative fluid ejection device such as a nasal spray device that may be used with the methods described herein is illustrated in a cross-sectional view, not to scale, in FIG. 5. The device 100 includes a cartridge body 102, having a fluid outlet nozzle 104 attached to the cartridge body 102. A fluid jet ejection cartridge 106 is disposed in the cartridge body 102. The fluid jet ejection cartridge 106 contains a fluid to be dispensed by the device 100. A logic board 108 is disposed in cartridge body 102 and is electrically connected via a logic board connector 110 to an ejection head 10 on the fluid jet ejection cartridge 106. The ejection head 10 includes plurality of nozzle holes 20 and associated fluid ejectors 22. A controller 114 is disposed on the logic board 108 or on the ejection head 10 for executing a control algorithm to control the ejection head 10 to modify the amount of fluid ejected from the ejection head using one or more of the methods described above. The device 100 may include a rechargeable battery 116 electrically connected to the logic board 108 for providing power to the ejection head 10. A power switch 118 is used to activate the device 100. A USB input 120 may also be included to reprogram the controller for use with different pharmaceutical fluids. An activation button 122 may be used to initiate delivery of fluid droplets on-demand by a user.

For the purposes of this specification and appended claims, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that can vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques.

While particular embodiments have been described, alternatives, modifications, variations, improvements, and substantial equivalents that are or can be presently unforeseen can arise to applicants or others skilled in the art. Accordingly, the appended claims as filed and as they can be amended are intended to embrace all such alternatives, modifications variations, improvements, and substantial equivalents.

## Claims

1. A method for delivering a predetermined amount of fluid from a fluid ejection head (10) **characterized in that** comprising:
measuring one or more parameters selected from the group consisting of fluid flow parameters and electrical parameters for the fluid ejection head (10) to provide one or more measured parameters;
calculating a fluid ejection offset value from the one or more measured parameters;
storing the fluid ejection offset value in a memory location on the fluid ejection head (10);
accessing the fluid ejection offset value by an ejection head controller (114); and
adjusting an amount of fluid ejected from the fluid ejection head (10) during a fluid ejection step using the fluid ejection offset value to provide the predetermined amount of fluid from the fluid ejection head (10).

2. The method of claim 1, wherein
the fluid flow parameters are selected from the group consisting of nozzle diameter, flow channel width (W1), ejection chamber area, nozzle plate layer thickness (T2), flow feature layer thickness (T1), flow channel length (L1), and combinations of two or more of the foregoing.

3. The method of claim 2, further comprising:
averaging one or more of the fluid flow parameters along a length (L) of each of the fluid ejection head (10) to provide a fluid flow parameter offset value for each of the fluid ejection head (10).

4. The method of claim 3, further comprising:
storing the fluid flow parameter offset value in the memory location on the fluid ejection head (10).

5. The method of claim 1, wherein
the electrical parameters are selected from the group consisting of heater sheet resistivity, heater sheet thickness, silicon nitride layer thickness, cavitation layer resistivity, cavitation layer thickness, and a combination of two or more of the foregoing.

6. The method of claim 5, further comprising:
averaging one or more of the electrical parameters to provide an electrical parameter offset value for the fluid ejection head (10).

7. The method of claim 6, further comprising:
Storing the electrical parameter offset value in the memory location on the fluid ejection head (10).

8. The method of claim 4, wherein
the electrical parameters are selected from the group consisting of heater sheet resistivity, heater sheet thickness, silicon nitride thickness, cavitation layer resistivity, cavitation layer thickness, and a combination of two or more of the foregoing.

9. The method of claim 8, further comprising:
averaging one or more of the electrical parameters along a length of the fluid ejection head (10) to provide an electrical parameter offset value.

10. The method of claim 9, further comprising:
combining the fluid flow parameter offset value and the electrical parameter offset value to provide the fluid ejection offset value.

11. The method of claim 1, wherein
the step of adjusting the amount of fluid ejected from the fluid ejection head (10) is selected from the group consisting of changing a dropcount of fluid droplets ejected during the fluid ejection step and changing a size of fluid droplets ejected during the fluid ejection step.

12. The method of claim 11, wherein
the size of fluid droplets ejected during the fluid ejection step is changed by a method selected from the group consisting of changing a fluid ejection temperature during the fluid ejection step, changing a firing pulse width of one or more fluid ejectors (22) during the fluid ejection step, changing a voltage to the fluid ejection head (10), changing a firing frequency of the one or more fluid ejectors (22) during the fluid ejection step, and a combination of two or more of the foregoing.

13. A method for ejecting a predetermined amount of fluid from a fluid ejection head (10) **characterized in that** comprising:
measuring one or more parameters selected from the group consisting of fluid flow parameters and electrical parameters for the fluid ejection head (10) to provide one or more measured parameters;
calculating a fluid ejection offset value from the one or more measured parameters;
storing the fluid ejection offset value in a memory location on the fluid ejection head (10);
accessing the fluid ejection offset value by an ejection head controller (114); and
adjusting a dropcount of fluid droplets ejected from the fluid ejection head (10) during a fluid ejection step using the fluid ejection offset value to provide the predetermined amount of fluid ejected.

14. The method of claim 13, wherein
the fluid flow parameters are selected from the group consisting of nozzle diameter, flow channel width (W1), ejection chamber area, nozzle plate layer thickness (T2), flow feature layer thickness (T1), flow channel length (L1), and combinations of two or more of the foregoing.

15. The method of claim 14, further comprising:
averaging one or more of the fluid flow parameters along a length (L) of the fluid ejection head (10) to provide a fluid flow parameter offset value.

16. The method of claim 15, further comprising:
storing the fluid flow parameter offset value in the memory location on the fluid ejection head (10).

17. The method of claim 16, wherein
the electrical parameters are selected from the group consisting of heater sheet resistivity, heater sheet thickness, silicon nitride layer thickness, cavitation layer resistivity, cavitation layer thickness, and a combination of two or more of the foregoing.

18. The method of claim 17, further comprising:
averaging one or more of the electrical parameters for the fluid ejection head (10) to provide an electrical parameter offset value.

19. The method of claim 18, further comprising:
combining the fluid flow parameter offset value and the electrical parameter offset value to provide the fluid ejection offset value.

20. The method of claim 19, wherein
the fluid ejection head (10) is a thermal fluid ejection head.
